# EUROPÄISCHE PATENTSCHRIFT

(11) **EP 1 990 065 B1**
(45) Veröffentlichungstag und Bekanntmachung des Hinweises auf die Patenterteilung: **16.12.2009**
(21) Anmeldenummer: 07107683.0
(22) Anmeldetag: 08.05.2007
(51) Int. Cl.: A61M 1/06

(54) **Milchpumpe**
Milk pump
Pompe à lait

(43) Veröffentlichungstag der Anmeldung: 12.11.2008
(60) Teilanmeldung: 08168743.6
(73) Patentinhaber: Trimed AG, 9495 Triesen (LI)
(72) Erfinder: Nüesch, Hansueli, 5453, Remetschwil (CH)
(74) Vertreter: Stocker, Kurt

(56) Entgegenhaltungen:
- WO-A-01/34226
- US-A- 4 929 229
- US-A- 5 843 029
- US-A1- 2003 204 164

## Beschreibung

Die Erfindung bezieht sich auf eine Milchpumpe nach dem Oberbegriff des Anspruches 1.

Eine derartige Milchpumpe ist beispielsweise aus der US-A-6,749,582 bekannt geworden. Dabei ist der Träger annähernd rohrförmig, wobei in seine obere Öffnung der Hubraum von oben her einsteckbar ist. Alternativ ist ein Schlauchanschluss für eine Fusspumpe vorgesehen. Soweit der Hubraum nur hineingesteckt wird, ist natürlich die Verbindung der beiden Teile sehr unzuverlässig. In ähnlicher Weise zeigt die US-A-6,652,484 eine Milchpumpe, bei der der zylindrische Hubraum einer Handpumpe in der Form einer Injektionsspritze abgeschraubt werden kann. Auch hier ist als Alternative ein Schlauchanschluss vorgesehen.

In beiden Fällen ist der Pumpenbetrieb etwas mühsam; im einen Fall wegen der blossen Steckverbindung und damit der Notwendigkeit die Saugmembrane nach oben hin zu bewegen, während der gekröpfte Betätigungshebel seitlich bewegt wird (was zu Reibungsverlusten führt und daher die Hand ermüden lässt); im anderen Fall, weil die Betätigung einer "Injektionsspritze" in zwei Richtungen zwei Hände für die Pumpe erfordert und ja überdies auch noch der Milchbehälter gehalten werden muss. Schlauchverbindungen dagegen sind wegen der Möglichkeit einer Verwicklung, der schlechten Reinigung wegen und auch wegen der Neigung der Kunststoffe solcher Schläuche zu Versprödung, Brüchigkeit etc. möglichst zu vermeiden.

Nun sei ein Blick darauf geworfen, warum man überhaupt eine solche lösbare Verbindung von Hubraum und Träger vornimmt. Ein Grund ist die Möglichkeit, die Pumpenteile separat zu reinigen, d.h. höheren Hygieneanforderungen zu entsprechen. Der andere Grund aber ist in beiden Fällen, dass man die Möglichkeit haben soll, wahlweise (wenigstens) zwei Arten von mit Muskelkraft betriebenen Pumpen einzusetzen. Es wurde oben bereits gesagt, dass dazu eine Schlauchverbindung nicht günstig ist; aber auch eine Schraubverbindung hat ihre Tücken: Zunächst ist bei der Herstellung, etwa im Spritzgussverfahren, die Entformung nicht ganz einfach, zumal das Gewinde wegen der nötigen Dichtung relativ lang sein muss. Dies führt zu höheren Kosten. Aber auch im praktischen Gebrauch ist das notwendige lange Schrauben umständlich und kann - bei falschem Ansatz der beiden Gewindeteile - leicht zum "Abwürgen" des Gewindes führen, was die Sache unbrauchbar macht. Ausserdem lässt eine Schraubverbindung nicht die Verwendung eines gekrümmten, etwa horizontal liegenden Hubraumes zu, wie er aus der WO 01/34226 bekannt geworden ist und allerdings der Hubraum in den kappenartigen Trägerteil integriert ist und daher keine separate Reinigung oder einen Austausch des Pumpensystems zulässt und auch hinsichtlich der Materialwahl Beschränkungen auferlegt, weil dieses so gewählt werden muss, dass es sowohl den Festigkeitsanforderungen genügt als auch zu keiner allzu grossen Reibung zwischen Kolben und Hubraum führt.

Eine Kombination eines lösbar befestigbaren Hubraumes mit einem Träger (der an sich beliebig ausgebildet sein kann) erlaubt es aber in vorteilhafter Weise auch, dass verschiedene Pumpenmodelle auf den Markt gebracht werden können, die alle denselben Trägerteil besitzen, der so in grösseren Stückzahlen kostengünstig gefertigt werden kann. Hier aber wäre eine Schraubverbindung ebenfalls nachteilig, weil sie mehr Arbeitsaufwand beim Zusammensetzen der Teile erfordert. Anderseits wurden bisher mit gutem Grund keine anderen Verbindungen als die genannten vorgeschlagen, weil die Pumpbewegung einerseits auf den Hubraum eine gewisse Längskraft in Bewegungsrichtung ausübt und daher die Verbindung möglichst fest sein sollte. Anderseits aber darf die Verbindung wieder nicht so sein, dass die Benützerin die Teile zum Reinigen kaum auseinanderbringt.

Der Erfindung liegt daher die Aufgabe zugrunde, eine Milchpumpe der eingangs genannten Art so auszubilden, dass sie beim Pumpen und beim Reinigen leicht zu handhaben ist, und dies gelingt erfindungsgemäss durch die kennzeichnenden Merkmale des Anspruches 1.

Durch die Verwendung einer mit einem Riegelelement verriegelbaren lösbaren Verbindung lassen sich die folgenden Vorteile erzielen:
1. die mindestens eine Wand des Hubraums ist zwar im Pumpbetrieb fest mit dem Träger verbunden, lässt sich aber mit einem einzigen Handgriff zum Reinigen lösen;
2. in der Fabrikation kann diese Wand oder der gesamte Hubraum einfach auf den Träger gesteckt werden, wo er dann verriegelt wird;
3. der Hubraum bzw. seine Umfangswand kann aus einem Material gefertigt werden, welches das Gleiten erleichtert, wogegen der Träger nur mehr eine Tragfunktion hat;
4. der Austausch des Hubraumes kann auch zu Reparaturzwecken leicht durchgeführt werden;
5. eine mit einem Riegelelement verriegelbare lösbare Verbindung lässt sich, beispielsweise im Spritzgussverfahren, leicht formen, was sich auch hinsichtlich der Herstellungskosten positiv auswirkt;
6. eine mit einem Riegelelement verriegelbare lösbare Verbindung hat keine engen Gewindegänge, die schwer zu reinigen sind.

Zur Erleichterung des Pumpens ist es vorteilhaft, wenn der Hubraum entsprechend dem Bewegungsbogen des schwenkbar gelagerten Hebels der Betätigungseinrichtung gekrümmt ist, weil dadurch die Bewegungsübertragung weitgehend reibungsfrei erfolgt.

In diesem Falle kann aber der Hubraum nur in einer einzigen, bestimmten Richtung an den Träger gesetzt werden, und deshalb ist es besonders bevorzugt, wenn eine Verdrehsicherung zur Sicherung der Übereinstimmung der Krümmung des Hubraumes und des Bewegungsbogens des Betätigungshebels vorgesehen ist. Eine günstige Ausführungsform wird später an Hand der Zeichnung beschrieben.

Wenn die Schnappverbindung an der gegen die zum Sammelbehälter führenden Öffnung gewandten Seite angeordnet ist, dann ist die Befestigung spielfrei gerade dort gesichert, wo der Übergang des Saugstromes leckfrei erfolgen soll.

Durch die Trennung von Träger und Hubraum (im Vergleich mit der Lösung nach der WO 01/34226) ist es nun möglich, sich den unterschiedlichen Erfordernissen besser anzupassen, weshalb es bevorzugt ist, wenn der Hubraum aus einem gegenüber dem Träger differierenden Material gebildet ist, insbesondere das Material des Hubraumes einen geringeren Reibungskoeffizienten besitzt als das Material des Trägers und/oder das Material des Trägers eine grössere Festigkeit bzw. Härte als das Material des Hubraumes aufweist. Beispielsweise kann der Hubraum aus Polyäthylen (PE) oder Polyamid (PA) gefertigt und/oder der Träger aus Polycarbonat (PC) oder Polypropylen (PP) hergestellt sein.

Weitere Einzelheiten der Erfindung ergeben sich an Hand der nachfolgenden Beschreibung von in der Zeichnung schematisch dargestellten Ausführungsbeispielen. Es zeigen:
Fig. 1 einen Vertikalschnitt durch ein erstes Ausführungsbeispiel einer erfindungsgemässen Milchpumpe etwa nach der Linie I-I der Fig. 5;
Fig. 2 eine perspektivische explodierte Darstellung der Milchpumpe nach Fig. 1, zu der die
Fig. 3 und 4 jeweils modular ansetzbare Einheiten für eine fixe Relativlage von Brustansatzstück und Betätigungshebel und für eine verstellbare Relativlage veranschaulichen;
Fig. 5 eine hintere Ansicht entsprechend dem Pfeil V der Fig. 2;
Fig. 6 eine der Fig. 2 ähnliche explodierte Darstellung einer anderen Ausführung oder der Pumpe nach Fig. 1 aber mit anderer Pumpeneinheit; und
Fig. 7 einen der Fig. 1 ähnlichen Schnitt durch die Ausführung nach Fig. 1, jedoch versetzt entlang der Linie VII-VII der Fig. 5.

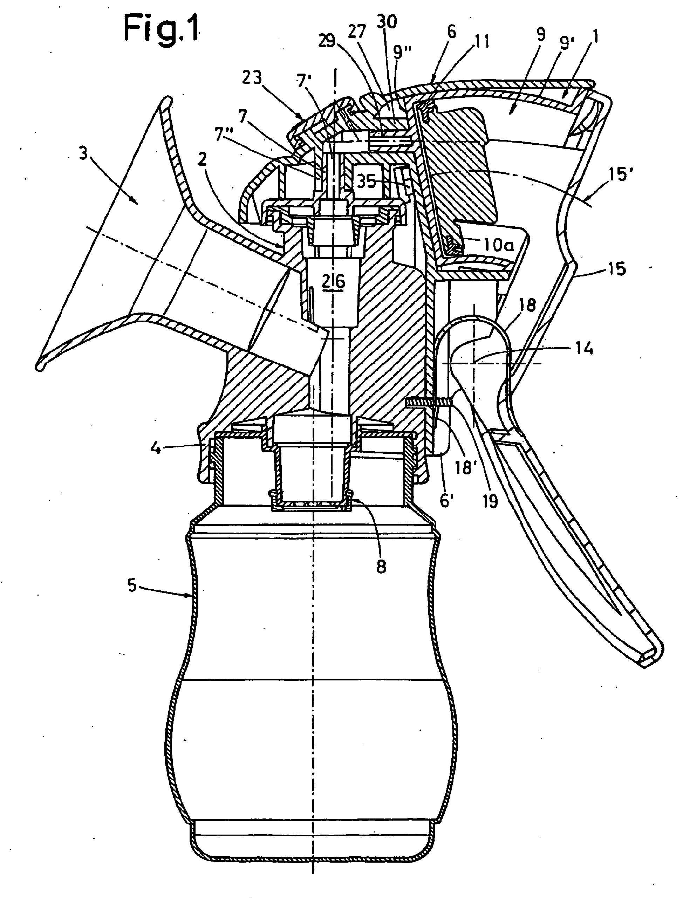

Die in Fig. 1 dargestellte Milchpumpe weist einen oberen Träger 6 mit einer Handpumpeneinheit 1 auf, der auf einen Verbindungsteil 2 aufgesteckt ist. Dazu besitzt der Verbindungsteil 2 einen nach oben ragenden Rohrstutzen 7 mit einer Verbindungsöffnung 7', auf welchen Stutzen 7 ein Rohrstutzen 7" des Trägers aufgesteckt ist. Die beiden Rohrstutzen 7, 7" sind leicht konisch, so dass eine dichte und feste Verbindung entsteht. Der Träger 6 könnte an sich auf die verschiedenste Art ausgebildet sein und beispielsweise aus einzelnen Holmen bestehen; bevorzugt aber ist er, wie dargestellt derart kappenartig ausgebildet, dass er in seinem Inneren die Handpumpeneinheit 1 geschützt aufnimmt.

Der Verbindungsteil 2 umfasst einerseits ein Brustansatzstück 3 oder Horn, anderseits einen Schraubteil 4 mit dem die Pumpe auf einen Milchsammelbehälter 5 aufschraubbar ist. Zwischen Milchsammelbehälter 5 und dem Verbindungsteil 2 liegt in bekannter Weise ein Rückschlagventil 8. Dieses Rückschlagventil 8 ist vorzugsweise so ausgebildet, wie dies in der in der US-A-6,270,474 beschrieben ist, deren Inhalt hier durch Bezugnahme als geoffenbart gelten soll.

An der Oberseite des Trägers 6 mag ein bekannter Reglerknopf 23 angebracht sein, der die Zufuhr von Falschluft regelt und damit auch die Mühe bei der Pumpbetätigung. Eine besonders bevorzugte Ausführung ist beispielsweise in den US-A-6,042,560 und 6,290,671 beschrieben, deren Inhalt hier durch Bezugnahme ebenfalls als geoffenbart gelten soll.

Um die Reinigung der nachstehend beschriebenen Handpumpeneinheit 1 nicht durch den Zutritt von Muttermilch noch zu erschweren, ist der Öffnung 7' ein Überlaufventil 26 vorgeordnet. Dieses Überlaufventil ist vorzugsweise so ausgebildet, wie es im EP-Patent Nr. 0 121 825 beschrieben ist, deren Inhalt hier durch Bezugnahme ebenfalls als geoffenbart gelten soll.

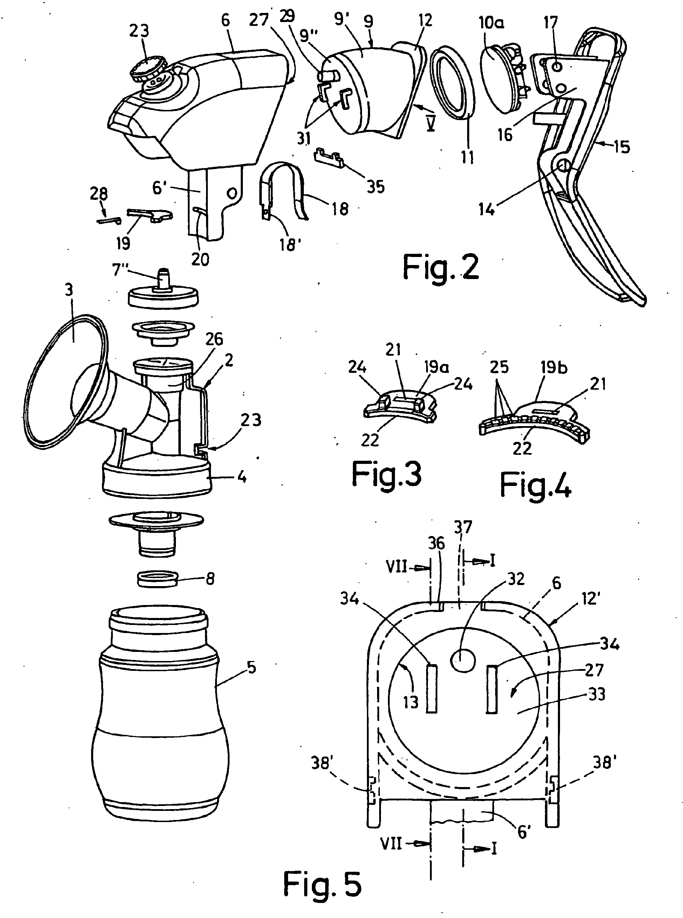

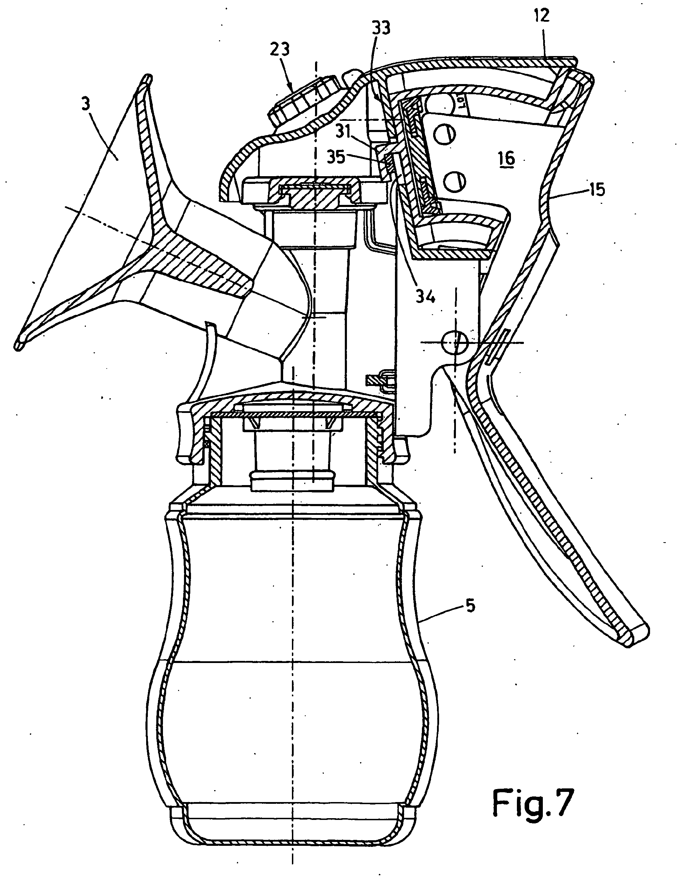

Innerhalb des kappenartigen Trägers 6 ist ein die Pumpeneinheit 1 mit einem Hubraum 9 aufnehmender Hohlraum 27 vorgesehen, wobei der Hubraum 9 von einer Umfangswand 9' umgeben und von einer Stirnwand 9" gegen die Öffnung 7' hin abgeschlossen ist. Dieser Hubraum 9 ist, wie besonders Fig. 2 zeigt, in einem kappenartig ausgebildeten Träger 6 mit im wesentlichen viereckigen Querschnitt mit abgerundeten Ecken (vgl. auch Fig. 5) geschützt eingesteckt und mit diesem lösbar verbunden. Da in dem Hubraum 9 ein Kolben 10a als Saugorgan mittels eines um einen Schwenkpunkt 14 schwenkbaren Betätigungsorganes bzw. (zweiarmigen) Hebels 15 hin- und herbewegt werden soll, muss bzw. soll der Bewegungsbogen 15' möglichst mit der Krümmung der Umfangswand 9' bzw. mit dem gekrümmten Verlauf des Hubraumes 9 übereinstimmen, weil sich andernfalls eine die pumpende Hand ermüdende Reibung ergibt. Um diese Übereinstimmung zu sichern, ist gemäss Fig. 2 zweckmässig ein mit dem Viereckquerschnitt des Trägers 6 übereinstimmender Schild 12 entweder in den ebenso ausgebildeten Hohlraum 27 des kappen-artigen Trägers 6 einsetzbar oder auf diese aufsteckbar (vgl. Fig. 7). Wie aus Fig. 1 ersichtlich, kann - bei sehr genauer Fertigung - auf ein solches Einsatzstück bzw. einen solchen Schild 12 auch verzichtet werden, obwohl er als Sicherung gegen Verdrehung des Hubraumes 9 bevorzugt ist.

Wie an Hand der als Abschlusselement dienenden Variante 12' des Rahmens oder Schilds in Fig. 5 ersichtlich ist, hat dieses eine Öffnung 13, durch welche ein mit einem von einem Dichtungsring 11 umgebenen Kolben 10a über Stecklöcher 17 verbindbarer Führungsteil 16 des Hebels 15 hindurch bewegbar ist. Dieser Hebel 15 steht beispielsweise unter der Rückholkraft einer Rückholfeder 18, die vorzugsweise als einfache U-förmige Blattfeder ausgebildet ist und mit einem Ende am Hebel 15 angreift (vgl. Fig. 1). Das andere Ende 18' der Feder 18 ist am Träger 6, gegebenenfalls aber auch am Verbindungsteil 2 befestigt. Dies kann mit einem Verbindungselement 19 geschehen, das beispielsweise einfach von einer Schraube gebildet sein mag. Dabei kann das Verbindungselement 19 gleich einem doppelten Zweck dienen, indem es auch den Träger 6 am Verbindungsteil 2 befestigt.

Fig. 2 zeigt eine bevorzugte Ausführung, bei welcher der nach unten ragende Trägerfuss 6' eine, z.B. schlitzartige, Öffnung 20 aufweist, durch die ein bogenförmiges Befestigungselement 19 wahlweise in der Ausführung 19a als Sperrorgan oder in der Ausführung 19b (vgl. Fig. 3, 4) als eine Relativdrehung des Hebels 15 gegenüber dem Behälter 5 und dem Träger 6 begrenzenden und damit teilweise zulassenden Begrenzungsorgan hindurchsteckbar ist. Dieses bogenförmige Befestigungselement 19a bzw. 19b besitzt jeweils einen Schlitz 21, durch welchen - nach dem Einschieben in den Schlitz 20 - hinter diesem Schlitz 20 das Ende 18' der Feder 18 eingeschoben werden kann und so das Verbindungselement 19 verriegelt. Der vordere Bogen 22 des jeweiligen Befestigungselementes 19 sitzt in einer Schlitzführung 23 (Fig. 2) des Verbindungsteiles 2, wobei im Falle des Befestigungselementes 19a Vorsprünge 24 die Endbegrenzungen des Schlitzes umfassen und so eine fixe Verbindung schaffen, welche keinerlei Relativdrehung von Verbindungsteil 2 und Träger zulässt.

Alternativ aber kann das Befestigungselement bzw. Begrenzungsorgan 19b in den Schlitz 20 eingeschoben und mittels des Endes 18' befestigt werden. Dieses Befestigungselement 19b ist breiter und besitzt Rasten 25, die eine begrenzte Verdrehbarkeit des Trägers 6 gegenüber dem Verbindungsteil 2 gestatten und die jeweilige Relativstellung verrasten. Dadurch ist eine Anpassung der Relativlage beim Pumpen an der linken oder rechten Brust möglich. Durch die Austauschbarkeit der Verbindungselemente 19a bzw. 19b ist es möglich, die Funktion der Pumpe den Wünschen der Benutzerin anzupassen bzw. können so auch leicht verschiedene Modelle der gezeigten Handpumpe erstellt werden, ohne die nötigen Formwerkzeuge zu stark zu verändern. Es versteht sich, dass diese Austauschbarkeit auch unabhängig davon denkbar ist, ob auch der Hubraum 9 oder mindestens eine Wand davon austauschbar ist oder nicht, so dass es sich hier an sich um eine gesonderte Erfindung handelt.

Es versteht sich, dass die beiden Teile 19a, 19b auf die verschiedenste Weise ausgebildet und entweder am Träger 6 bzw. seinem Fuss 6' - um mit den Begrenzungsflächen bzw. einer Gegenrast (vgl. Blattfeder 28 mit Gegenrastnoppe in Fig. 1) am Verbindungsteil 2 zusammenzuwirken - oder am Verbindungsteil 2 vorgesehen werden können - um mit Begrenzungsflächen bzw. einer Gegenrast am Trägerfuss 6' zusammenzuwirken. Auch können die Rasten statt an der Oberseite etwa an der dem Verbindungsteil (oder dem Träger) gegenüberliegenden Stirnseite des Teiles 19b ausgebildet sein. Letztlich liesse sich gegebenenfalls aber auch auf Rasten 25 verzichten und nur eben weiter voneinander beabstandete Vorsprünge (entsprechend den Vorsprüngen 24) vorsehen, was aber weniger bevorzugt ist.

Bezüglich der eben erwähnten Austauschbarkeit des Hubraumes 9 oder mindestens einer Wand 9' und/oder 9" davon sei nun folgendes erläutert.

Wie aus den Fig. 2 und 1 oder (besser 7) hervorgeht, ragt von der Stirnwand 9" des Hubraumes 9 einerseits ein Verbindungsrohrstutzen 29 vor. Gemäss Fig. 1 ist dieser Rohrstutzen 29 in einen Gegenstutzen 30 am Träger 6 steckbar. Da beide Stutzen wieder etwas konisch sind, ergibt sich eine abgedichtete Steckverbindung, welche Leckagen verhindert.

Von der Stirnwand 9" (Fig. 2) ragen aber auch zwei Teile einer Verriegelungseinrichtung, die im dargestellten Ausführungsbeispiel von zwei Haken 31 gebildet sind. Beim Einschieben des Hubraumes 9 in den Hohlraum 27 des Trägers 6 tritt einerseits der Rohrstutzen 29 durch eine kreisrunde Öffnung 32 einer Aufnahmeplatte 33 (Fig. 5, 7) des Trägers 6, anderseits durchsetzen die Haken 31 je einen Schlitz 34 der Platte 33. Nun kann ein Riegel 35, z.B. ein die Stirnwand 9" gegen die Platte 33 pressender Keilriegel, zwischen die Haken 31 geschoben werden, wie besonders aus Fig. 7 ersichtlich ist. Es versteht sich aber, dass dies nur eines von vielen möglichen Beispielen für eine Verrieglung ist, wobei der Fachmann für den Bau von Verrieglungen zahlreiche Abwandlungen kennt. Beispielsweise könnte die Stirnwand 9" seitliche Fortsätze haben, an denen beispielsweise ein Verriegelungsexzenter anzugreifen vermag, um diese Wand 9" gegen die Platte 33 zu pressen. In jedem Falle aber genügt ein Riegel zweierlei Bedingungen:
1. er ist leicht und rasch einsetzbar und auch wieder entfernbar, und
2. er widersteht grossen Kräften in Bewegungsrichtung des Saugorganes bzw. im konkreten Fall entlang des Bewegungsbogens 15'.

Es ist klar, dass der Ort der Verriegelung, wie oben bereits angedeutet - verschieden gewählt werden kann. Wird er im Bereich der Umfangswand 9' gewählt, dann geht aber in der Regel Platz für den Hubraum verloren. Wird der Ort der Verriegelung am (in der Zeichnung) rechten, offenen Ende des Trägers 6 gewählt, dann wäre der dichte Anschluss zur Öffnung 7' nur erschwert gesichert. Deshalb ist es bevorzugt, wenn die verriegelbare Verbindung 31, 35 an der gegen die zum Behälter 5 führenden Öffnung 7' gewandten Seite angeordnet ist, wie dies aus der Zeichnung zu ersehen ist. Es versteht sich ferner, dass es an sich auch denkbar wäre, die Wand 33 als Stirnwand des Hubraumes 9 auszubilden und nur dessen Umfangswand 9' mit dieser so gebildeten Stirnwand zu verriegeln. In diesem Falle kann sich aber die Abdichtung erschweren bzw. wäre dann sich eine etwa kreisringförmige Dichtung an der Stirnseite dieser Umfangswand 9' nötig, wobei die Reinigung u.U. erschwert sein mag.

Es ist aus der EP 1 231 955 bekannt, den Träger kappenartig auszubilden, allerdings zu einem anderen Zweck, denn dort bildet er gleichzeitig die Umfangsbegrenzung des Hubraumes. Dies hat - ausser der erschwerten Reinigung des Hubraumes - zur Folge, dass man bei der Herstellung stets einen Kompromiss eingehen muss, denn entweder besteht die Wand des Trägers aus einem festen Trägermaterial, welches aber zu mehr Reibung des Kolbens und zu einer erschwerten Betätigung führt, oder man verwendet ein gleitfreundliches Material, das aber dann weniger Formstabilität besitzt. Durch die erfindungsgemäss vorgesehene Trennung der beiden Aufgaben kann nun für beide Anforderungen ein Optimum erzielen, kurz gesagt, indem die Umfangswand des Hubraumes aus einem gegenüber dem Träger differierenden Material gebildet ist. Dabei wird man zweckmässig so vorgehen, dass das Material des Hubraumes einen geringeren Reibungskoeffizienten besitzt als das Material des Trägers und/oder das Material des Trägers eine grössere Festigkeit bzw. Härte als das Material des Hubraumes aufweist. In der Praxis wird man vorteilhaft den Hubraum aus Polyäthylen (PE) oder Polyamid (PA) fertigen und/oder den Träger aus Polycarbonat (PC) oder Polypropylen (PP) herstellen.

Was die schon erwähnte Fig. 5 angeht, so ist aus dieser auch die Befestigung des Schildes 12 am äusseren Ende seines Hohlraumes 27 ersichtlich. Dazu wird er mittels einer, vorzugsweise elastischen, Umfangsspange 12' (Fig. 5, 7) an der Trägerkappe befestigt, d.h. die Spange wird über den Rand des kappenartigen Trägers 6' geschoben und hält bei der Ausführung nach Fig. 1 den Schild 12, bei der Ausführung nach Fig. 7 einen Rand 38 einer topfförmigen Membrane 39, auf die noch eingegangen wird. Jedenfalls kann durch die von einer Kreisform abweichende Form des Hohlraumes 27 (vgl. auch Fig. 5) für eine Verdrehsicherung gesorgt werden, weil dann der gekrümmte Hubraum 9 nur so eingesetzt werden kann, dass seine Krümmung mit der Bogenbewegung 15' (Fig. 1) übereinstimmt.

Die Spange 12' besitzt an ihrer Oberseite eine Schlitzöffnung 36, durch welche ein auch aus der Trägerausführung nach Fig. 7 ersichtlicher Lappen oder Vorsprung 37 eingreift. Wenn also diese Spange 12 aus einem leicht elastischen Material geformt ist, so kann sie ohne weiteres über den Lappen 37 gezogen werden. Die unteren Ränder der Spange 12' können durch Rastnoppen 38' festgehalten werden.

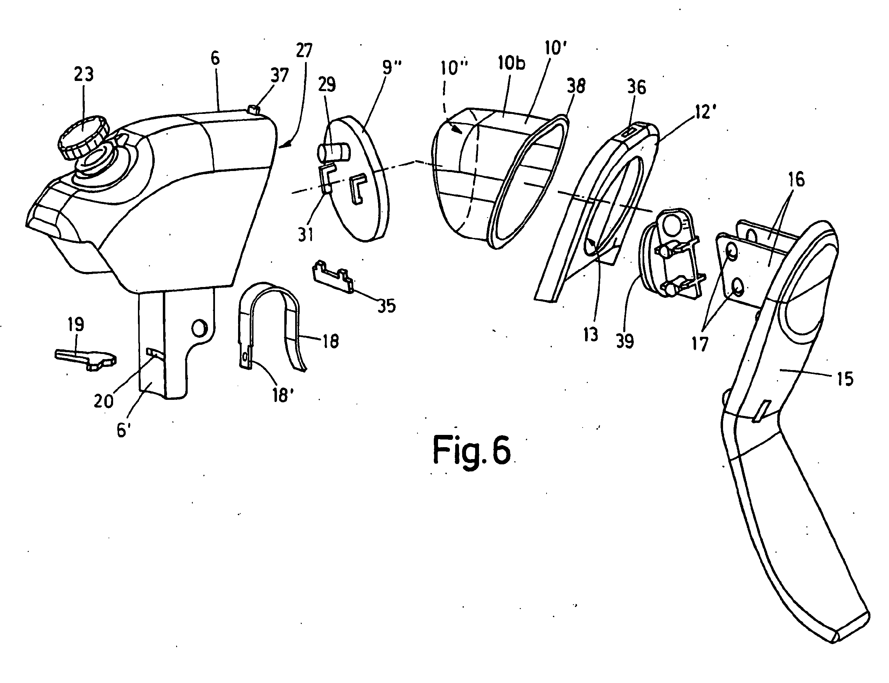

An Hand der Fig. 6 soll gezeigt werden, dass die Erfindung die Herstellung unterschiedlicher Modelle mit geringem Aufwand erlaubt. Für gleiche Teile werden dabei dieselben Bezugszeichen wie in den vorigen Figuren verwendet.

Die Teile an der linken und rechten Seite der Fig. 6 stimmen mit den in Fig. 2 an diesen Seiten gezeigten Teilen überein. Unterschiedlich ist, dass an Stelle des Kolbens 10a (Fig. 2) eine etwa topfförmige Membrane 10b mit einer Umfangswand 10' und einer vorderen, strichliert angedeuteten Stirnwand 10" vorgesehen ist, vorzugsweise so, wie dies in der Europäischen Patentanmeldung EP 071013.46.0 beschrieben ist. An dieser Stirnwand ist ein die kreisrunde Öffnung 13 (vgl. Fig. 5) durchgreifender Anschlussteil 39 befestigt, der seinerseits mit dem Führungsteil 16 des Hebels 15 über die Stecklöcher 17 mittels Steckstiften verbunden ist. Nun könnte an sich auch für die Topfmembrane 10b ein gekrümmter Zylinderraum entsprechend dem Hubraum 9 vorgesehen werden. Es wurde aber oben bereits auf den besonderen Vorteil der lösbaren Anbringung des Hubraumes 9 hingewiesen, der u.a. in der Möglichkeit der Anpassung des Kunststoffes an die verschiedenen Reibungsverhältnisse liegt. Bei Verwendung einer Membrane 10b braucht ja aber auf den Reibungskoeffizienten des Materials gar keine Rücksicht genommen werden, und deshalb genügt es, wenn an Stelle des ganzen Hubraumes 9 gemäss Fig. 2 nur die Stirnwand 9" zur Abdeckung der Schlitze 34 bzw. für die Verbindung der Rohrstutzen 29, 30 (vgl. Fig. 1, 5, 6) eingesetzt wird. Dagegen steht der gesamte Hohlraum 27 des Trägers 6 zur Unterbringung der Topfmembrane 10b zur Verfügung, d.h. sie kann im Querschnitt grösser als der Kolben 10a sein und daher auch eine grössere Pumpleistung erbringen. Zur Befestigung der Membrane 10b am Träger 6 ist die bereits an Hand der Fig. 5 beschriebene, vorzugsweise elastische Spange 12' vorgesehen, welche den Rand 38 der Membrane 10b am Rande des Trägers 6 und dessen Hohlraumes 27 festklemmt.

Bezugszeichenliste
1 Handpumpeneinheit 2 Verbindungsteil
3 Brustansatzstück 4 Schraubteil
5 Behälter 6 Träger mit Fuss 6'
7 Rohrstutzen 7' Öffnung in 7
7" Rohrstutzen von 6 8 Rückschlagventil
9 Hubraum 9' Umfangswand von 9
9" Stirnwand von 9 10 Saugorgan (10a oder 10b)
11 Dichtungsring von 10 12 Rahmen, Schild; 12' = Spange
13 Öffnung von 12 14 Schwenkpunkt von 15
15 Betätigungseinrichtung bzw. Hebel 15' Bewegungsbogen v. 15 16 Führungsteil an 15 17 Stecklöcher v. 16
18 Rückholfeder 19 Verbindungselement (19a, 19b)
20 Öffnung in 6' 21 Schlitz v. 19
22 Bogen v. 19 23 Schlitzführung v. 2
24 Vorsprünge v. 19a 25 Rasten v. 19b
26 Überlaufventil 27 Hohlraum in 6
28 Blattfeder mit Noppe f. 19b 29 Rohrstutzen v. 9"
30 Gegenstutzen an 6 31 Verriegelungshaken
32 kreisrunde Öffnung in 33 33 Platte v. 6
34 Schlitze in 33 für 31 35 Riegel
36 Schlitzöffnung 37 Lappen
38 Membranrand 39 Anschlussteil
38' Rastnoppen

## Patentansprüche

1. Milchpumpe mit einer Handpumpeneinheit (1) oberhalb einer zu einem Behälter (5) führenden Öffnung (7') an einem Träger (6), welche Einheit (1) einen im wesentlichen von einer Umfangswand (9') und einer stirnseitigen Abschlusswand (9") umschlossenen, an ein Brustansatzstück (3) über den Träger lösbar anschliessbaren Hubraum (9) und ein Saugorgan (10a, 10b) aufweist, das darin mittels einer manuellen Betätigungseinrichtung (15), wie einem Hebel (15) - vorzugsweise einem zweiarmigen, in einem Mittelbereich (bei 19) schwenkbar gelagerten Hebel (15) - hin- und herbewegbar ist, **dadurch gekennzeichnet, dass** wenigstens eine Wand (9'; 9"), vorzugsweise wenigstens die Abschlusswand (9"), des Hubraumes (9) über eine mit einer Riegelanordnung (31, 35) verriegelbare lösbare Verbindung am Träger (6) leicht austauschbar befestigt ist.

2. Milchpumpe nach Anspruch 1, **dadurch gekennzeichnet, dass** wenigstens eines der folgenden Merkmale vorgesehen ist:
a) eine Rückholeinrichtung (18) für die Betätigungseinrichtung (15) zum Rückholen in eine vorbestimmte Ausgangslage;
b) der Träger (6) überdeckt haubenartig den sich annähernd horizontal erstreckenden Hubraum (9);
c) der zum Behälter (5) führenden Öffnung (7') ist ein Überlaufventil (26) vorgeschaltet.

3. Milchpumpe nach Anspruch 1 oder 2, **dadurch gekennzeichnet, dass** der mit seiner Umfangswand (9') austauschbare Hubraum (9) entsprechend dem Bewegungsbogen (15') des schwenkbar gelagerten Hebels (15) der Betätigungseinrichtung gekrümmt ist, und dass vorzugsweise eine Verdrehsicherung (6, 12, 27) zur Sicherung der Übereinstimmung der Krümmung des Hubraumes (9) und des Bewegungsbogens (15') des Betätigungshebels (15) vorgesehen ist, und gegebenenfalls der Träger (6) eine von einer Kreisform abweichende, insbesondere eckige, z.B. viereckige, Hohlform (27) aufweist, welche den Hubraum (9), allenfalls mit in die Ecken passendem Einsatzstück (12), aufnimmt.

4. Milchpumpe nach einem der vorherigen Ansprüche, **dadurch gekennzeichnet, dass** die verriegelbare Verbindung (31, 35) an der gegen die zum Behälter (5) führenden Öffnung (7') gewandten Seite angeordnet ist.

5. Milchpumpe nach einem der vorherigen Ansprüche, **dadurch gekennzeichnet, dass** mindestens die Umfangswand (9') des Hubraumes (9) aus einem gegenüber dem Träger (6) differierenden Material gebildet ist, insbesondere das Material des Hubraumes (9) einen geringeren Reibungskoeffizienten besitzt als das Material des Trägers (6) und/oder das Material des Trägers (6) eine grössere Festigkeit bzw. Härte als das Material des Hubraumes (9) aufweist, und dass vorzugsweise der Hubraum (9) aus Polyäthylen (PE) oder Polyamid (PA) gefertigt ist und/oder dass der Träger (6) aus Polycarbonat (PC) oder Polypropylen (PP) hergestellt ist.

6. Milchpumpe nach einem der Ansprüche 1 bis 5, **dadurch gekennzeichnet, dass** das Saugorgan einen im Hubraum (9) entlang bewegbaren Kolben (10a) aufweist.

7. Milchpumpe nach einem der Ansprüche 1 bis 5, **dadurch gekennzeichnet, dass** das Saugorgan eine im Hubraum (9) bewegbare topfförmige Membrane (10b) aufweist.

8. Milchpumpe nach einem der vorherigen Ansprüche, **dadurch gekennzeichnet, dass** zwischen Träger (6) und wenigstens einer Wand (9") des Hubraumes (9) eine Dichtung vorgesehen ist, und dass vorzugsweise diese Dichtung als eingepasste Konus-Steckverbindung zwischen einem konischen Rohransatz (29) am einen Teil und einem diesen aufnehmenden, vorzugsweise ebenfalls konischen, Kanal (30) am anderen Teil ausgebildet ist.

9. Milchpumpe nach einem der vorherigen Ansprüche, **dadurch gekennzeichnet, dass** ein Verbindungsteil (2) mit einer Verbindung zu der zum Behälter (5) führenden Öffnung (7') versehen ist, und dass dieser Verbindungsteil (2) und/oder der an ihm befestigbare Träger (6) ein ein Lösen erlaubendes Befestigungselement (19) - alternativ für ein gegen eine Drehung um die Achse der zum Behälter (5) führenden Öffnung (7') sicherndes, lösbares Sperrorgan (19a), oder für ein eine solche Drehung begrenzendes und damit teilweise zulassendes Begrenzungsorgan (19b) - aufweist.

## Claims

1. Milk pump comprising a manual pumping unit (1) above an opening (7'), which leads to a recipient (5), of a carrier (6), said unit (1) including a swept volume space (9), which is substantially enclosed by a peripheral wall (9') and a frontal closing wall (9") and is releasably connectable via said carrier to a breast joining piece (3), and a suction member (10a, 10b), which is movable therein to and fro by manual actuation means (15), such as a lever (15), preferably a two-armed lever (15) pivotally supported in a middle region (at 19), **characterised in that** at least one wall (9', 9"), preferably at least said closing wall (9"), of said swept volume space (9) is exchangeably fastened to said carrier (6) by a releasable connection to be latched by a latching arrangement (31, 35).

2. Milk pump according to claim 1, **characterised in that** at least one of the following characteristics is provided:
a) return motion means (18) for said actuation means (15) for returning it into a predetermined initial position;
b) said carrier (6) covers in a hood-like manner said swept volume space (9), which extends approximately horizontally;
c) an overflow valve (26) is pre-posed to said opening (7') leading to the recipient (5).

3. Milk pump according to claim 1 or 2, **characterised in that** said swept volume space (9), which is exchangeable with its peripheral wall (9'), is curved in correspondence with the arc of motion (15') of the pivotally supported lever (15) of said actuation means, and that preferably a twisting safeguard arrangement (6, 12, 27) is provided for safeguarding the coincidence of the curvature of said swept volume space (9) and said arc of motion (15') of the actuation lever (15), and that optionally said carrier (6) has a hollow shape (27) deviating from a circular shape, particularly an angular, e.g. a four-cornered, shape, in some case comprising an insert piece (12) fitting into the corners.

4. Milk pump according any of the preceding claims, **characterised in that** said connection to be latched (31, 35) is arranged at that side, which is turned towards said opening (7'), which leads to the recipient (5).

5. Milk pump according any of the preceding claims, **characterised in that** at least the peripheral wall (9') of said swept volume space (9) is formed of a material that is different from that of said carrier (6), and that in particular the material of said swept volume space (9) has a smaller friction coefficient than the material of said carrier (6) and/or that the material of said carrier (6) has a higher strength or hardness than the material of said swept volume space (9), and that preferably the swept volume space (9) is made of polyethylene (PE) or of polyamide (PA) and/or that the carrier (6) is made of polycarbonate (PC) or of polypropylene (PP).

6. Milk pump according any of claims 1 to 5, **characterised in that** said suction member comprises a piston (10a) moveable along said swept volume space (9).

7. Milk pump according any of claims 1 to 5, **characterised in that** said suction member comprises a cup-shaped membrane (10b) moveable within said swept volume space (9).

8. Milk pump according any of the preceding claims, **characterised in that** a sealing means is provided between said carrier (6) and at least one wall (9") of said swept volume space (9), and that preferably this sealing means is formed as a fitting cone plug connection between a conical pipe joint (29) on one part and a channel (30), that is preferably conical too, on the other part which receives the same.

9. Milk pump according any of the preceding claims, **characterised in that** a connection part (2) is provided with a connection to said opening (7'), which leads to the recipient (5), and that this connection part (2) and/or the carrier (6) fixed to it comprises a fastening element (19) permitting release, alternatively for a releasable locking element (19a), which secures against rotation about the axis of said opening (7'), which leads to the recipient (5), or for a limiting element (19b) which limits such rotation, thus partially allowing it.

## Revendications

1. Pompe à lait comprenant une unité de pompe manuelle (1) au-dessus d'une ouverture (7'), qui conduit à un récipient (5), à un support (6), ladite unité (1) incluant une cylindrée (9), qui est sensiblement enserrée par une paroi périphérique (9') et une paroi de fermeture frontale (9") et qui peut être reliée à travers le support (6) d'une manière détachable à une pièce d'embout de buste (3), et un organe d'aspiration (10a, 10b), qui y est mobile avant arrière par un moyen manuel d'actuation (15), comme un levier (15), de préférence un levier (15) à deux bras étant logé d'une manière pivotante dans une zone médiane (chez 19), **caractérisée en ce, qu**'au moins une paroi (9', 9"), de préférence au moins la paroi de fermeture (9"), de la cylindrée (9) est montée d'une façon facilement échangeable au support (6) par une connexion détachable, capable d'être verrouillée par une disposition de verrouillage (31, 35).

2. Pompe à lait selon la revendication 1, **caractérisée en ce, qu**'au moins une des caractéristiques suivantes est prévue :
a) un moyen de rappel (18) pour le moyen d'actuation (15) pour le rappeler à une position initiale ;
b) le support (6) couvre la cylindrée (9), qui s'étend approximativement en direction horizontale, d'une façon similaire à un capot ;
c) une soupape de trop-plein (26) est montée en série à l'ouverture (7'), qui conduit au récipient (5).

3. Pompe à lait selon la revendication 1 ou 2, **caractérisée en ce, que** la cylindrée (9), échangeable avec sa paroi périphérique (9'), est arquée en correspondance avec l'arc de mouvement (15') du levier (15) du moyen d'actuation logé d'une manière pivotante, et que de préférence un moyen de sécurité contre une torsion (6, 12, 27) est prévu pour assurer la coïncidence de la courbure de la cylindrée (9) et de l'arc de mouvement (15') du levier d'actuation (15), et que, le cas échéant, le support (6) a une forme creuse (27) déviant d'une forme circulaire, particulièrement une forme angulée, par exemple quadrangulaire, qui reçoit la cylindrée (9), le cas échéant avec un insert (12) adapté aux coins.

4. Pompe à lait selon une quelconque des revendications précédentes, **caractérisée en ce, que** la connexion capable d'être verrouillée (31, 35) est disposée au côté tourné vers l'ouverture (7'), qui conduit au récipient (5).

5. Pompe à lait selon une quelconque des revendications précédentes, **caractérisée en ce, qu**'au moins la paroi périphérique (9') de la cylindrée (9) est formée d'un matériau, qui diffère par rapport à ceci du support (6), où particulièrement le matériau de la cylindrée (9) a un coefficient de frottement plus bas que le matériau du support (6) et/ou le matériau du support (6) a une résistance mécanique ou une dureté plus grande que le matériau de la cylindrée (9), et que de préférence la cylindrée (9) est fabriquée du polyéthylène (PE) ou du polyamide (PA) et/ou que le support (6) est fabriqué du polycarbonate (PC) ou du polypropylène (PP).

6. Pompe à lait selon une quelconque des revendications 1 à 5, **caractérisée en ce, que** l'organe d'aspiration comprend un piston (10a) mobile le long de et dans la cylindrée (9).

7. Pompe à lait selon une quelconque des revendications 1 à 5, **caractérisée en ce, que** l'organe d'aspiration comprend une membrane en forme de pot (10b) mobile dans la cylindrée (9).

8. Pompe à lait selon une quelconque des revendications précédentes, **caractérisée en ce, qu**'un joint d'étanchéité est prévu entre le support (6) et au moins une paroi (9") de la cylindrée (9), et que de préférence ce joint d'étanchéité est formée comme un raccord affleuré mâle et femelle à cône entre une tubulure conique (29) à une partie et un conduit (30), de préférence conique aussi, qui reçoit ce dernier, à l'autre partie.

9. Pompe à lait selon une quelconque des revendications précédentes, **caractérisée en ce, qu**'une partie de connexion (2) est pourvue d'une connexion vers l'ouverture (7'), qui conduit au récipient (5), et que cette partie de connexion (2) et/ou le support (6) y fixable comprend un élément des fixage (19) permettant l'enlèvement, ce qui est alternativement pour un organe de blocage (19a) détachable, qui arrête contre une rotation autour de l'axe de l'ouverture (7'), qui conduit au récipient (5), ou pour un organe de délimitation (19b), qui délimite une telle rotation et ainsi la permet partiellement.
